# EUROPEAN PATENT APPLICATION

(11) **EP 4 249 601 A1**
(43) Date of publication of application: **27.09.2023**
(21) Application number: 21894508.7
(22) Date of filing: 08.11.2021
(51) Int. Cl.: C12Q 1/02, C12Q 1/6851, C12Q 1/686, G01N 33/50, G01N 33/574, G01N 33/68

(54) **METHOD FOR ASSISTING DETERMINATION ON ADAPTABILITY OF ESOPHAGEAL CANCER TO ENDOSCOPIC THERAPY, METHOD FOR DETERMINING ADAPTABILITY OF ESOPHAGEAL CANCER TO ENDOSCOPIC THERAPY, METHOD FOR COLLECTING DATA FOR USE IN DETERMINATION ON ADAPTABILITY OF ESOPHAGEAL CANCER TO ENDOSCOPIC THERAPY, METHOD FOR DIAGNOSING ADAPTABILITY OF ESOPHAGEAL CANCER TO ENDOSCOPIC THERAPY, IN VITRO METHOD FOR ASSISTING DETERMINATION ON ADAPTABILITY OF ESOPHAGEAL CANCER TO ENDOSCOPIC THERAPY, METHOD FOR TESTING ON ADAPTABILITY OF ESOPHAGEAL CANCER TO ENDOSCOPIC THERAPY, METHOD FOR DIAGNOSING ADAPTABILITY OF ESOPHAGEAL CANCER TO ENDOSCOPIC THERAPY, DIAGNOSIS KIT, AND METHOD FOR TREATING ESOPHAGEAL CANCER**

(30) Priority: 17.11.2020 JP 2020190857
(71) Applicant: Public University Corporation Nagoya City University, Mizuho-ku Nagoya-shi Aichi 467-8601 (JP)
(72) Inventor: SHIMURA Takaya, Nagoya-shi, Aichi 467-8601 (JP); OKUDA Yusuke, Nagoya-shi, Aichi 467-8601 (JP); KATAOKA Hiromi, Nagoya-shi, Aichi 467-8601 (JP)
(74) Representative: Papula Oy
(86) International application number: PCT/JP2021/041056
(87) International publication number: WO 2022/107634

(57) **Abstract**

The present invention provides a versatile method which has a high diagnostic accuracy rate on the suitability of esophageal cancer for endoscopic therapy and enables the simple and objective determination of the suitability or unsuitability for endoscopic therapy.

In the method according to one aspect of the present invention, an expression level of at least one or more selected from the group consisting of CXCL2 and VEGFA in a sample collected from a subject is measured, and the expression level is compared with a reference value; the diagnostic kit according to one aspect of the present invention comprises a reagent for measuring at least one or more selected from the group consisting of the expression level of CXCL2 in the sample and the expression level of VEGFA in the sample; in the method for treating esophageal cancer according to one aspect of the present invention, the degree of vertical invasion of esophageal cancer in a subject is determined by employing a method in which an expression level of at least one or more selected from the group consisting of CXCL2 and VEGFA in a sample collected from a subject is measured and the expression level is compared with a reference value.

## Description

### [Technical Field]

The present invention relates to a method for assisting determination on suitability of esophageal cancer for endoscopic therapy, a method for determining suitability of esophageal cancer for endoscopic therapy, a method for collecting data for use in determination on suitability of esophageal cancer for endoscopic therapy, a method for diagnosing suitability of esophageal cancer for endoscopic therapy, an in vitro method for assisting determination on suitability of esophageal cancer for endoscopic therapy, a method for testing on suitability of esophageal cancer for endoscopic therapy, a method for diagnosing suitability of esophageal cancer for endoscopic therapy, a diagnostic kit, and a method for treating esophageal cancer.

Priority is claimed on Japanese Patent Application No. 2020-190857 filed November 17, 2020, the entire contents of which are incorporated herein by reference.

### [Background Art]

Esophageal cancer can be classified into superficial esophageal cancer and advanced esophageal cancer based on the difference in the degree of vertical invasion. For superficial esophageal cancer, endoscopic therapy can be selected as a treatment method in a case where cancer cells remain within the mucosa. On the other hand, when cancer cells invade the submucosa from the muscularis mucosae across the mucosa, or when cancer cells invade the vessel, surgical operation or chemoradiotherapy is selected for superficial esophageal cancer.

Surgical treatment methods are highly invasive compared to endoscopic therapy, and the incidence of postoperative complications is also high in surgical operations. Therefore, it is necessary to accurately diagnose the degree of vertical invasion of cancer cells and appropriately determine the suitability for endoscopic therapy. This is to prevent additional surgical resection after endoscopic therapy has been performed once, and to prevent selecting surgical resection or the like despite the possibility of complete cure with endoscopic therapy.

A method for determining the degree of vertical invasion of esophageal cancer includes image diagnoses such as an MRI test, a CT test, and endoscopic observation. However, it is difficult to accurately diagnose differences in the degree of invasion of esophageal cancer within the esophageal wall using imaging techniques such as an MRI test and a CT test. Therefore, morphological diagnosis by endoscopic observation is performed as a standard diagnostic method for diagnosing the degree of vertical invasion of superficial esophageal cancer (Non-Patent Document 1).

### [Citation List]

### [Non-Patent Document]

[Non-Patent Document 1]
ESD/EMR guideline for esophageal cancer (Gastroenterological Endoscopy Vol. 62(2), Feb. 2020, 221-271)

### [Summary of Invention]

### [Technical Problem]

In esophageal cancer, since the possibility of lymph node metastasis increases in a case where cancer cells invade the esophageal vessels and submucosa, it is required to confirm the degree of vertical invasion of cancer cells within the esophageal wall at the micro level and to determine the suitability for endoscopic therapy.

However, in a case where the degree of vertical invasion of esophageal cancer cells at the micro level is determined by endoscopic observation, it is necessary for the examiner to capture minute changes of the esophageal wall. Therefore, in morphological diagnosis by endoscopic observation, it is often difficult to determine the suitability for endoscopic therapy. In actual clinical practice, the diagnostic accuracy rate on the suitability for endoscopic therapy is about 60% to about 70%. In addition, since morphological diagnosis by endoscopic observation is empirical analog diagnosis, conventional methods have room for improvement in objectivity and are not versatile.

The present invention provides a versatile technique which has a high diagnostic accuracy rate on the suitability of esophageal cancer for endoscopic therapy and enables the simple and objective determination of the suitability or unsuitability for endoscopic therapy.

### [Solution to Problem]

A first aspect of the present invention has the following embodiments [1] to [5].
[1] A method for assisting determination on suitability of esophageal cancer for endoscopic therapy, the method comprising measuring the expression level of at least one or more selected from the group consisting of CXCL2 and VEGFA in a sample collected from a subject, and comparing the expression level with a reference value.
[2] The method according to [1], in which the expression levels of both CXCL2 and VEGFA in the sample are measured.
[3] The method according to [1] or [2], in which the sample is at least one or more selected from the group consisting of serum, a serum exosome, and tumor tissue.
[4] The method according to any of [1] to [3], in which determining whether or not esophageal cancer has invaded the esophageal vessel or submucosa is assisted.
[5] A diagnostic kit used to determine the suitability of esophageal cancer for endoscopic therapy, the diagnostic kit comprising a reagent kit that is used for measuring at least one or more selected from the group consisting of the expression level of CXCL2 in a sample and the expression level of VEGFA in a sample.

A second aspect of the present invention further has embodiments described in the following «1» to «7» in addition to the embodiments described in [1] to [5] above.
«1» A method for determining suitability of esophageal cancer for endoscopic therapy, the method comprising measuring the expression level of at least one or more selected from the group consisting of CXCL2 and VEGFA in a sample collected from a subject, and comparing the expression level with a reference value.
«2» The method according to «1», in which the expression levels of both CXCL2 and VEGFA in the sample are measured.
«3» The method according to «1» or «2», in which the sample is at least one or more selected from the group consisting of serum, a serum exosome, and tumor tissue.
«4» The method according to any of «1» to «3», in which it is determined whether or not esophageal cancer has invaded the esophageal vessel or submucosa.
«5» A method for treating esophageal cancer, in which the degree of vertical invasion of esophageal cancer in a subject is determined using the method of any of «1» to «4», and endoscopic therapy is selected in a case where esophageal cancer is predicted not to have invaded the esophageal vessel.
«6» A method for treating esophageal cancer, in which the degree of vertical invasion of esophageal cancer in a subject is determined using the method of any of «1» to «4», and endoscopic therapy is selected in a case where esophageal cancer is predicted not to have invaded the submucosa.
«7» A method for treating esophageal cancer, in which the degree of vertical invasion of esophageal cancer in a subject is determined using the method of any of «1» to «4», and surgical therapy is selected in a case where esophageal cancer is predicted to have invaded the esophageal vessel or esophageal cancer is predicted to have invaded the submucosa.

### [Effects of Invention]

According to the present invention, a versatile technique which has a high diagnostic accuracy rate on the suitability of esophageal cancer for endoscopic therapy and enables the simple and objective determination of the suitability or unsuitability for endoscopic therapy is provided.

### [Description of Drawings]

FIG. 1 is a diagram explaining the relationship between the degree of vertical invasion of esophageal cancer and the suitability of esophageal cancer for endoscopic therapy and surgical operation.
FIG. 2 is a flowchart explaining an outline of the analysis of the experimental examples.
FIG. 3 is an ROC curve created from the measurement results of CXCL2 and VEGFA in serum.
FIG. 4 is a diagram explaining the results in a case of using CXCL2 singly in the ROC curve of FIG. 3.
FIG. 5 is a diagram explaining the results in a case of using CXCL2 and VEGFA concurrently in the ROC curve of FIG. 3.
FIG. 6 is a diagram showing the results of measuring the expression level of CXCL2 mRNA in esophageal cancer tumor tissue.
FIG. 7 is a diagram showing the results of measuring the expression level of VEGFA mRNA in esophageal cancer tumor tissue.

### [Description of Embodiments]

The following terms used herein have the meanings set forth in this paragraph.

"Esophageal cancer" refers to a malignant tumor that develops in the esophagus (the part of the digestive tract that extends from the pharynx to the stomach). Esophageal cancer is pathologically classified into squamous cell carcinoma, adenocarcinoma, mucoepidermoid carcinoma, or the like, but is not limited to these.

A "primer" means a nucleotide that forms a complementary pair with either or both of DNA and RNA.

A "mutant" means a natural mutant due to polymorphism, mutation, or the like, and a mutant containing deletion, substitution, addition, or insertion of one or more bases or amino acid residues.

A "derivative" means a derivative labeled with a fluorophore, a radioactive isotope, or the like, a modified nucleotide with an organic functional group, a nucleotide that has undergone rearrangement of bases, saturation of a double bond, deamination, substitution of an oxygen molecule with a sulfur molecule, or the like, and the like. However, a derivative is not limited to these examples.

A "subject" means primates such as humans and chimpanzees, mammals including rodents such as mice and rats, and the like; pets such as dogs and cats; and livestock such as cattle, horses, sheep, and goats.

A "person to be subjected" means a human as a subject.

Actions identified by the terms "test", "evaluation", and "examination" do not include medical actions by physicians (for example, an action of diagnosing a human disease, an action of treating human diseases, and the like) in countries where medical actions are excluded from the object of patent.

"Sensitivity" means the value of (the number of true positives)/((the number of true positives) + (the number of false negatives)).

"Specificity" means (the number of true negatives)/((the number of true negatives) + (the number of false positives)).

"To" indicating a numerical range means that the numerical values before and after it are included as lower and upper limits.

### <Method for assisting determination on suitability of esophageal cancer for endoscopic therapy>

The method of the present invention is a method for assisting determination on suitability of esophageal cancer for endoscopic therapy.

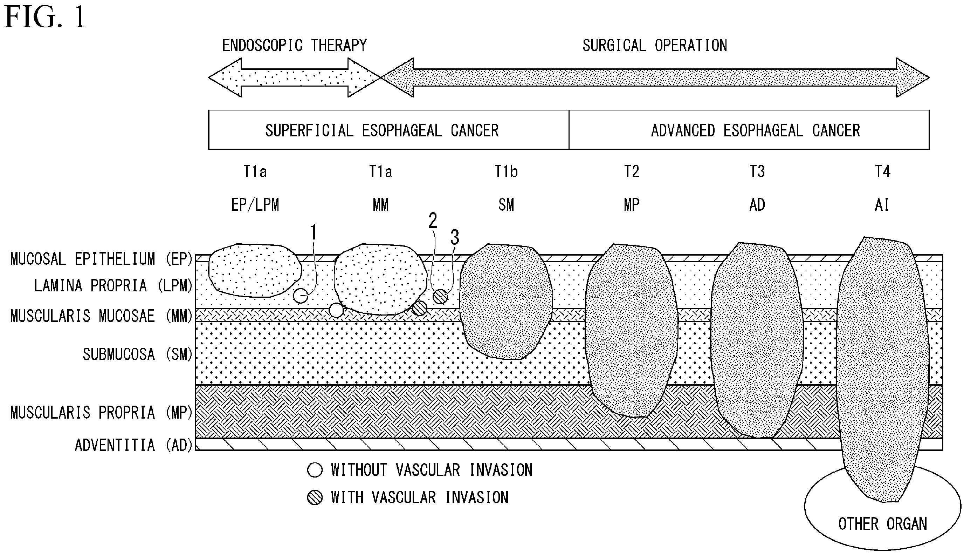

As shown in FIG. 1, esophageal cancer is classified into superficial esophageal cancer and advanced esophageal cancer according to the depth of the degree of vertical invasion from the mucosal epithelium to the adventitia. For advanced esophageal cancer, surgical operation is the standard therapy in a case where curative resection is possible. On the other hand, among superficial esophageal cancers (T1a or T1b) in which cancer invasion is limited to the submucosa, intramucosal cancer (T1a) can be completely cured by endoscopic therapy and is suitable for endoscopic therapy.

In recent years, in particular, endoscopic resection has been selected even for cancers in which cancer cells have not penetrated into minute vessels among cancers that have invaded the muscularis mucosae (T1a MM). For example, in FIG. 1, reference numeral 1 indicates a vessel not invaded by cancer cells. In a case where cancer cells have not invaded the vessel, endoscopic therapy can be selected. On the other hand, in FIG. 1, reference numeral 2 indicates a vessel invaded by cancer cells, and reference numeral 3 indicates cancer cells that have invaded an esophageal vessel. Surgical operation is recommended in a case where cancer cells have invaded a vessel.

In the method of the present invention, by measuring the concentration of at least one or more selected from the group consisting of CXCL2 and VEGFA in a sample, the degree of vertical invasion in superficial esophageal cancer is predicted, and determining or diagnosing the suitability for endoscopic therapy or the suitability for surgical operation is assisted.

In particular, in the present invention, it is possible to predict whether or not esophageal cancer has invaded the esophageal vessel or submucosa, and to assist in determining the suitability or unsuitability for endoscopic therapy.

In the method of the present invention, the expression level of at least one or more selected from the group consisting of CXCL2 and VEGFA in a sample collected from a subject is measured, and the expression level is compared with a reference value.

Examples of the sample include body fluid of a subject and tumor tissue (biopsy tissue) collected from the subject. Examples of body fluid include body fluids such as blood, serum, milk, urine, saliva, lymph, cerebrospinal fluid, amniotic fluid, tears, sweat, rhinorrhea, and fecal fluid; and exosomes contained in these body fluids. However, body fluids are not limited to these examples. A treated liquid obtained by pretreatment such as removing unnecessary components from each of the body fluids, or a culture medium obtained by culturing cells contained in each of the body fluids may also be used. One kind thereof may be used singly or two or more thereof them may be used concurrently.

Among these, serum and a serum exosome are preferable because the effects of the invention can be easily confirmed.

CXCL2 is a type of CXC chemokine, which is a cytokine. CXCL2 is an abbreviation for "Chemokine (C-X-C motif) ligand 2". CXCL2 is also sometimes referred to as Gro-β, GRO2, or MIP-2α.

VEGFA is a type of vascular endothelial cell growth factor. VEGFA is an abbreviation for "Vascular Endothelial Growth Factor-A". VEGFA is also sometimes referred to as vascular endothelial growth factor A, or simply VEGF.

In the method of the present invention, it is preferable to measure the expression levels of both CXCL2 and VEGFA in the sample because of higher sensitivity. In addition, in the method of the present invention, the expression level of a mutant or a derivative of each of CXCL2 and VEGFA may be measured.

The expression level of CXCL2 or VEGFA may be the expression level of DNA encoding CXCL2 or VEGFA, may be the expression level of RNA encoding CXCL2 or VEGFA, and may be the protein expression level of CXCL2 or VEGFA.

For example, in a case where the protein concentration of CXCL2 or VEGFA in a sample is measured as its expression level, various protein measurement methods such as ELISA, Multiplex, and the like can be used for the sample. The method for measuring the protein concentration is not limited to ELISA and Multiplex, and various other protein measurement methods can be used.

In measuring the protein expression level, the expression level may be standardized using a protein whose expression level is constantly stable in the sample as a normalization factor.

The method for measuring the DNA expression level and the RNA expression level is not particularly limited as long as the concentration of DNA or RNA can be measured. Examples of a method for measuring DNA concentration include PCR, but various other measurement methods can be used. In addition, examples of a method for measuring RNA concentration include reverse transcription PCR and qPCR, but various other RNA measurement methods can be used. The expression level of DNA and the expression level of RNA may be calculated based on the cutoff value of the PCR cycle number.

In measuring the DNA expression level or the RNA expression level, the expression level may be standardized using other DNA or RNA other than that encoding CXCL2 or VEGFA and whose expression level is constantly stable in the sample, as a normalization factor.

Next, in the method of the present invention, the measured expression level of CXCL2 or VEGFA is compared with a reference value. By comparing at least one or more values selected from the group consisting of the expression level of CXCL2 and the expression level of VEGFA with a reference value, the degree of vertical invasion of cancer cells in the esophageal wall at the micro level can be predicted with an excellent diagnostic accuracy rate. Therefore, the possibility of appropriately determining the suitability for endoscopic therapy is higher than in the prior art.

As the reference value, a reference value at which cancer cells are suspected of invading the submucosa across the muscularis mucosae in the esophagus in a case of being no less than this value can be considered. As another reference value, a reference value at which cancer cells are suspected of invading the esophageal vessels in a case of being no less than this value can be considered.

In defining the reference value, the expression levels of CXCL2 and VEGFA in subjects which are suitable for endoscopic therapy may be used as a reference. Usually, the reference values are the expression levels of CXCL2 and VEGFA in a subject which is suitable for endoscopic therapy. The reference value used for discrimination can be appropriately set according to the subject's age, sex, collecting method, type of sample, desired sensitivity, specificity, diagnostic accuracy rate, and the like.

Details will be described later in experimental examples, but in a case where the expression level of CXCL2 or VEGFA in the sample is lower than the reference value, it can be predicted that cancer cells have not invaded either the esophageal submucosa or the esophageal vessels, and it can be determined that the endoscopic therapy is preferably selected. On the other hand, in a case where the expression level of CXCL2 or VEGFA in the sample is no less than the reference value, it can be predicted that cancer cells have invaded the submucosa across the muscularis mucosae in esophagus, or cancer cells have invaded the esophageal vessels, and it can be determined that surgical operation or chemoradiotherapy instead of endoscopic therapy is preferably selected.

In the above-described method for assisting determination on suitability of esophageal cancer for endoscopic therapy, the expression level of at least one or more selected from the group consisting of CXCL2 and VEGFA in a sample is measured, and the expression level is compared with a reference value. As shown in Examples below, the presence or absence of invasion of esophageal cancer into the submucosa or the vessels can be predicted from the concentration of CXCL2 or VEGFA in the sample. Therefore, according to the method of the present invention, it can be determined whether a subject is suitable for endoscopic therapy or for surgical operation, with an excellent diagnostic accuracy rate.

In addition, the method of the present invention is a digital method based on test values, different from empirical analog diagnosis such as morphological diagnosis, and is a method that has excellent objectivity and is versatile.

It can be said that the method for assisting determination on suitability of esophageal cancer for endoscopic therapy described above is a method for collecting data for use in determination on suitability of esophageal cancer for endoscopic therapy.

It can be said that the method for assisting determination on suitability of esophageal cancer for endoscopic therapy described above is a method for diagnosing suitability of esophageal cancer for endoscopic therapy.

It can be said that the method for assisting determination on suitability of esophageal cancer for endoscopic therapy described above is a method for testing on suitability of esophageal cancer for endoscopic therapy.

It can be said that the method for assisting determination on suitability of esophageal cancer for endoscopic therapy described above is an in vitro method for assisting determination on suitability of esophageal cancer for endoscopic therapy.

It can be said that the method for assisting determination on suitability of esophageal cancer for endoscopic therapy described above is a method for diagnosing suitability of esophageal cancer for endoscopic therapy.

It can be said that the method for assisting determination on suitability of esophageal cancer for endoscopic therapy described above is a method for determining suitability of esophageal cancer for endoscopic therapy.

It can be said that the method for assisting determination on suitability of esophageal cancer for endoscopic therapy described above is a method for examining suitability of esophageal cancer for endoscopic therapy.

### <Diagnostic kit>

The diagnostic kit of the present invention is a diagnostic kit used for determining suitability of esophageal cancer for endoscopic therapy. The diagnostic kit of the present invention comprises a reagent kit that is used for measuring at least one or more selected from the group consisting of the expression level of CXCL2 in a sample and the expression level of VEGFA in a sample.

Examples of the reagent kit for measuring the expression level of each DNA of CXCL2 and VEGFA include those having at least primers specific to each DNA of CXCL2 and VEGFA. The reagent kit for measuring the expression level of DNA may further comprise at least one selected from the group consisting of DNA extraction reagents and PCR enzymes. The DNA extraction reagent is not particularly limited as long as it can extract DNA from the sample.

Examples of the reagent kit for measuring the expression level of each RNA of CXCL2 and VEGFA include those having at least primers specific to each mRNA of CXCL2 and VEGFA. By performing RT-PCR, qPCR, or the like after reverse transcription reaction is performed from mRNA of CXCL2 or VEGFA and specific primers, the expression level of RNA of CXCL2 or VEGFA can be measured.

In addition, the reagent kit for measuring the expression level of RNA may further comprise at least one selected from the group consisting of an RNA extraction reagent and a reverse transcriptase. The RNA extraction reagent is not particularly limited as long as it can extract RNA from the sample. The reverse transcriptase is not particularly limited as long as it can be used for normal RT-PCR or qPCR.

The reagent kit for measuring the expression levels of CXCL2 and VEGFA proteins is not particularly limited as long as it can measure the concentrations of CXCL2 and VEGFA in a sample. Examples thereof include reagent kits for performing various analytical methods such as ELISA, western blotting, and the like.

In a case of measuring the expression level in exosomes, the diagnostic kit of the present invention may further comprises at least one or more selected from the group consisting of an exosome extraction kit for extracting exosomes from a sample, an RNA extraction kit for extracting RNA from exosomes, and a protein extraction kit for extracting proteins from exosomes.

In a case where blood is collected from a subject, the diagnostic kit of the present invention may further comprise an injection needle, a blood collection tube, and the like.

### (Treatment method)

The treatment method and treatment policy for esophageal cancer will be described. In countries where the action of diagnosing or treating humans is not patentable, the method of the present invention does not apply to a treatment method.

In countries where the action of diagnosing or treating humans is patentable, the method of the present invention is used to predict the degree of vertical invasion of esophageal cancer in a subject, and in a case where it is predicted that esophageal cancer has not invaded the submucosa, endoscopic therapy is preferably selected. In addition, in a case where it is predicted that esophageal cancer has not invaded the esophageal vessels, endoscopic therapy is preferably selected. On the other hand, in a case where it is predicted that esophageal cancer has invaded the esophageal submucosa, or in a case where it is predicted that esophageal cancer has invaded the esophageal vessels, surgical therapy is preferably selected. In particular, in a case where it is predicted that esophageal cancer has invaded the esophageal vessels, it is considered that surgical therapy is preferably selected regardless of the degree of vertical invasion of esophageal cancer. Treatment method is not particularly limited as long as it is approved or authorized in the country where the treatment is performed. Various treatment techniques can be applied as long as the treatment is expected to have a curative effect on cancer.

### [Examples]

Hereinafter, the present invention will be described in more detail below with reference to experimental examples, but the present invention is not limited to the following experimental examples.

### <Explanation of abbreviations>

n: number of cases
AUC: Area Under the ROC Curve
95% CI: 95% confidence interval

### <Analysis of protein expression level>

### (Overview of analysis)

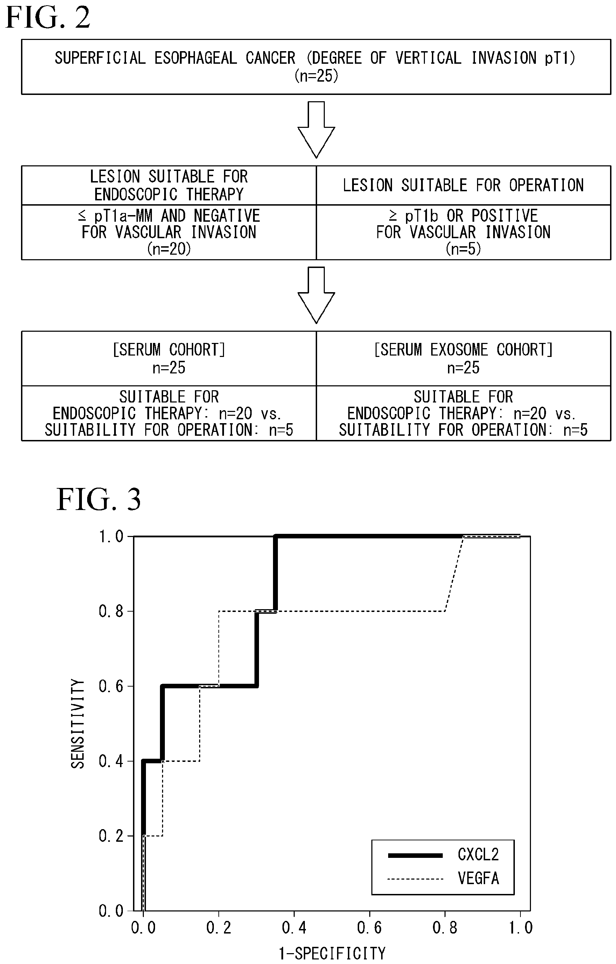

Among 44 cases of esophageal cancer that were resected endoscopically or surgically and in which samples were collected before treatment, 25 cases of superficial esophageal cancer with the degree of vertical invasion of T 1 were analyzed (FIG. 2). Among these 25 cases, based on the results of postoperative histopathological diagnosis, esophageal cancer with the degree of vertical invasion of T1a without lymph node metastasis and vascular invasion (negative for vascular invasion) was defined as "a lesion suitable for endoscopic therapy", and esophageal cancer with lymph node metastasis or vascular invasion (positive for vascular invasion), or esophageal cancer with the degree of vertical invasion of T1b or more, was defined as "a lesion suitable for operation". Endoscopic therapy is recommended for "a lesion suitable for endoscopic therapy". In addition, surgical operation is recommended for "a lesion suitable for operation".

Furthermore, in addition to the analysis of normal serum proteins, exosomes were extracted from serum and the analysis of serum exosomal proteins was performed (FIG. 2). The analysis of serum proteins and serum exosomal proteins was performed, and concentrations of CXCL2, VEGFA, and nine other angiogenic factors were measured by ELISA and Multiplex. Although the results are not shown here, the nine other factors were specifically FGF-basic, P1GF, Angiopoietin-1, PDGF-AA, Thrombospondin 2, Angiogenin, MEP1a, CXCL3, and Endostatin.

Compared with these nine other factors, a significant difference was observed between "a lesion suitable for endoscopic therapy" and "a lesion suitable for operation" in each concentration of CXCL2 and VEGFA. Table 1 shows the measurement results of CXCL2 and VEGFA.

**[Table 1]**

| | | Lesion suitable for endoscopic therapy | Lesion suitable for operation | P value |
|---|---|---|---|---|
| CXCL2 | Serum (pg/ml) | 222.1 ± 107.9 | 656.2 ± 592.9 | 0.003 |
| | Serum exosome (pg/ml) | 77.1 ± 36.9 | 737.6 ± 1362.4 | 0.030 |
| VEGFA | Serum (pg/ml) | 67.1 ± 44.8 | 141.6 ± 92.0 | 0.014 |
| | Serum exosome (pg/ml) | 18.6 ± 18.4 | 51.3 ± 43.5 | 0.014 |

### (Construction of ROC curve)

A significant increase in CXCL2 and VEGFA concentrations in serum and a serum exosome was observed in "a lesion suitable for operation" compared to "a lesion suitable for endoscopic therapy" (Table 1). On the other hand, no significant difference was observed between the two groups for the nine other factors.

FIG. 3 shows ROC curves created from the measurement results of CXCL2 and VEGFA in serum. In the analysis using this ROC curve, the AUC was 0.860 for CXCL2 in serum and 0.755 for VEGFA in serum, which was a good result (Table 2).

**[Table 2]**

| | AUC |
|---|---|
| CXCL2 | 0.860 |
| VEGFA | 0.755 |

### (Usage Example 1)

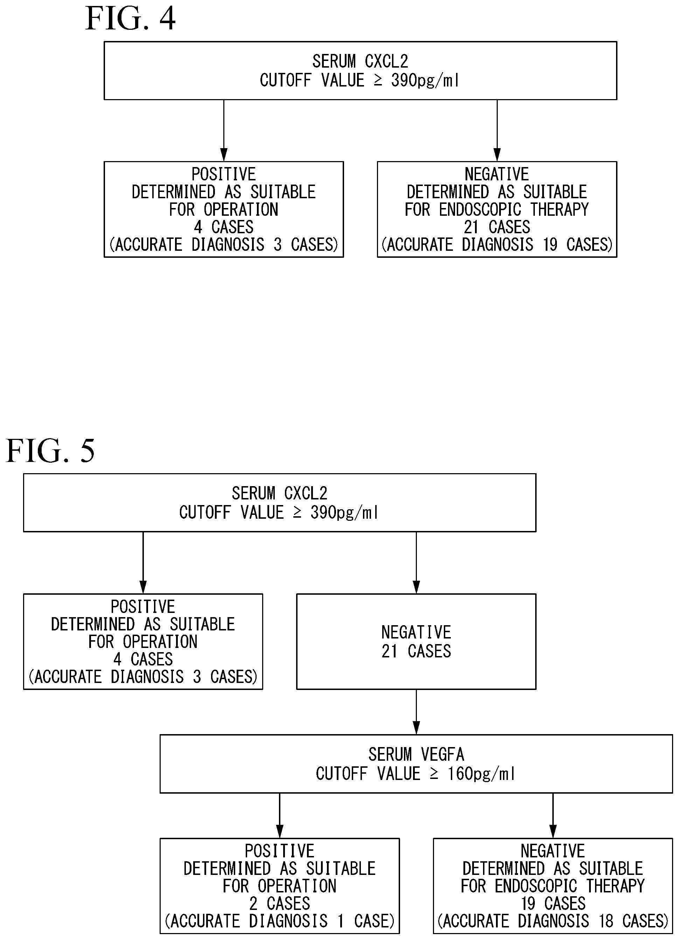

Two Usage Examples are presented for determining the treatment policy for superficial esophageal cancer. First, the expression level of CXCL2 protein in serum was used singly. Specifically, using the ROC curve shown in FIG. 3, CXCL2 ≥ 390 pg/ml was set as the cutoff value for the lesion suitable for operation. At this time, among all cases, 4 cases were classified as suitability for operation, and 21 cases were classified as suitability for endoscopic therapy (FIG. 4).

In fact, among all 25 cases, 5 cases were lesion suitable for operation and 20 cases were a lesion suitable for endoscopic therapy, and thus the sensitivity was 60%, the specificity was 95%, and the diagnostic accuracy rate was 88% in Usage Example 1 (Table 3) in a case where suitability for operation was defined as "positive" and suitability for endoscopic therapy was defined as "negative".

**[Table 3]**

| | CXCL2 singly in serum | | |
|---|---|---|---|
| | Positive | Negative | Total |
| Suitability for operation | 3 | 2 | 5 |
| Suitability for | 1 | 19 | 20 |
| endoscope | | | |
| Total | 4 | 21 | 25 |

### (Usage Example 2)

Next, the expression level of CXCL2 protein and the expression level of VEGFA protein in serum were used concurrently. In Usage Example 2, CXCL2 ≥ 390 pg/ml was defined as "suitability for operation" (positive), and in a case of satisfying VEGFA ≥ 160 pg/ml, it was defined as "suitability for operation" (positive) even if CXCL2 < 390 pg/ml. On the other hand, in a case of satisfying CXCL2 < 390 pg/ml and VEGFA < 160 pg/ml, it was defined as "suitability for endoscopic therapy" (negative) (FIG. 5). In Usage Example 2, the sensitivity was 80%, the specificity was 90%, and the diagnostic accuracy rate was 88% (Table 4).

**[Table 4]**

| | CXCL2 in serum + VEGFA in serum concurrently | | |
|---|---|---|---|
| | Positive | Negative | Total |
| Suitability for operation | 3 + 1 | 1 | 5 |
| Suitability for endoscope | 1 + 1 | 18 | 20 |
| Total | 6 | 19 | 25 |

Both Usage Examples 1 and 2 showed a higher diagnostic accuracy rate than the morphological diagnosis such as existing endoscopic diagnosis. In the future, by using the method of the present invention concurrently in addition to existing endoscopic diagnosis and morphological diagnosis, further improvement of the diagnostic accuracy rate will be expected.

In Usage Example 1 or Usage Example 2, CXCL2 ≥ 390 pg/ml and VEGFA ≥ 160 pg/ml were used as cutoff values for the lesion suitable for operation, but these cutoff values, that is, reference values can be changed appropriately. In particular, they can be optionally changed and set according to the desired sensitivity, specificity, and diagnostic accuracy rate.

### <Analysis of expression level of RNA>

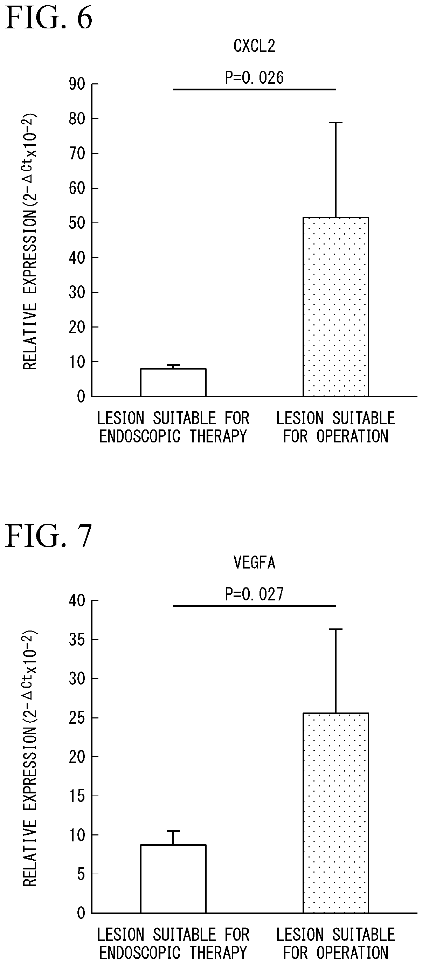

RNAs were extracted from biopsy tumor tissues collected from all 25 cases, and the expression of CXCL2 and VEGFA in the tumor tissue was analyzed by RT-PCR. The expression level of RNA was calculated based on the difference in the cutoff value of the PCR cycle number (difference from the endogenous factor). As a result, RNA expression of CXCL2 and VEGFA in tumor tissue was significantly higher in the lesion suitable for operation as compared in the lesion suitable for endoscopic therapy (FIGs. 6 and 7).

From these results, it was suggested that the expressions of CXCL2 and VEGFA were increased and proteins of CXCL2 and VEGFA whose expression level was increased were secreted into the blood in superficial esophageal cancers having high invasive capacity such that among early esophageal cancer, cancer cells invaded the submucosa, invaded the esophageal vessel, or could cause lymph node metastasis.

As described above, by measuring the expression level of at least one or more selected from the group consisting of CXCL2 and VEGFA, the degree of progression of superficial esophageal cancer, particularly whether or not cancer cells have invaded the esophageal vessels or whether or not cancer cells have invaded the esophageal submucosa can be predicted, and determining and diagnosing whether the cancer is suitable for endoscopic therapy or suitable for surgical operation can be assisted.

### [Industrial Applicability]

According to the present invention, a versatile method which has a high diagnostic accuracy rate on the suitability of esophageal cancer for endoscopic therapy and enables the simple and objective determination of the suitability or unsuitability for endoscopic therapy can be provided.

### [Reference Signs List]

1 Vessel not invaded by cancer cells
2 Vessel invaded by cancer cells
3 Cancer cells having invaded the vessel

## Claims

1. A method for assisting determination on suitability of esophageal cancer for endoscopic therapy, the method comprising:
measuring an expression level of at least one or more selected from the group consisting of CXCL2 and VEGFA in a sample collected from a subject, and comparing the expression level with a reference value.

2. A method for determining suitability of esophageal cancer for endoscopic therapy, the method comprising:
measuring an expression level of at least one or more selected from the group consisting of CXCL2 and VEGFA in a sample collected from a subject, and comparing the expression level with a reference value.

3. A method for collecting data for use in determination on suitability of esophageal cancer for endoscopic therapy, the method comprising:
measuring an expression level of at least one or more selected from the group consisting of CXCL2 and VEGFA in a sample collected from a subject, and comparing the expression level with a reference value.

4. A method for diagnosing suitability of esophageal cancer for endoscopic therapy, the method comprising:
measuring an expression level of at least one or more selected from the group consisting of CXCL2 and VEGFA in a sample collected from a subject, and comparing the expression level with a reference value.

5. A method for testing on suitability of esophageal cancer for endoscopic therapy, the method comprising:
measuring an expression level of at least one or more selected from the group consisting of CXCL2 and VEGFA in a sample collected from a subject, and comparing the expression level with a reference value.

6. An in vitro method for assisting determination on suitability of esophageal cancer for endoscopic therapy, the method comprising:
measuring an expression level of at least one or more selected from the group consisting of CXCL2 and VEGFA in a sample collected from a subject, and comparing the expression level with a reference value.

7. A method for diagnosing suitability of esophageal cancer for endoscopic therapy, the method comprising:
measuring an expression level of at least one or more selected from the group consisting of CXCL2 and VEGFA in a sample collected from a subject, and comparing the expression level with a reference value.

8. The method according to any one of Claims 1 to 7,
wherein expression levels of both CXCL2 and VEGFA in the sample are measured.

9. The method according to any one of Claims 1 to 8,
wherein the sample is at least one or more selected from the group consisting of serum, a serum exosome, and tumor tissue.

10. The method according to any one of Claims 1 to 9,
wherein the method assists determining whether or not esophageal cancer has invaded an esophageal vessel or a submucosa.

11. A diagnostic kit that is used to determine suitability of esophageal cancer for endoscopic therapy, the diagnostic kit comprising:
a reagent kit that is used for measuring at least one or more selected from the group consisting of an expression level of CXCL2 in a sample and an expression level of VEGFA in a sample.

12. A method for treating esophageal cancer,
wherein a degree of vertical invasion of esophageal cancer in a subject is determined using the method according to any one of Claims 1 to 10, and
endoscopic therapy is selected in a case where esophageal cancer is predicted not to have invaded an esophageal vessel.

13. A method for treating esophageal cancer,
wherein a degree of vertical invasion of esophageal cancer in a subject is determined using the method according to any one of Claims 1 to 10, and
endoscopic therapy is selected in a case where esophageal cancer is predicted not to have invaded a submucosa.

14. A method for treating esophageal cancer,
wherein a degree of vertical invasion of esophageal cancer in a subject is determined using the method according to any one of Claims 1 to 10, and
surgical therapy is selected in a case where esophageal cancer is predicted to have invaded an esophageal vessel or esophageal cancer is predicted to have invaded a submucosa.
